(19) Europäisches Patentamt
European Patent Office
Office européen des brevets

(11) Veröffentlichungsnummer: **0 135 897**
**A2**

# (12) EUROPÄISCHE PATENTANMELDUNG

(21) Anmeldenummer: **84110981.2**

(22) Anmeldetag: **14.09.84**

(51) Int. Cl.⁴: **C 07 C 31/44**, C 07 C 27/02

(30) Priorität: **27.09.83 JP 176882/83**

(43) Veröffentlichungstag der Anmeldung: **03.04.85 Patentblatt 85/14**

(84) Benannte Vertragsstaaten: **AT BE CH DE FR GB IT LI NL**

(71) Anmelder: **NIHON TOKUSHU NOYAKU SEIZO K.K., No.4, 2-chome, Nihonbashi Honcho Chuo-ku, Tokyo 103 (JP)**

(72) Erfinder: **Shiokawa, Kozo, 210-6, Shukugawara Tama-ku, Kawasaki-shi Kanagawa-ken (JP)**
Erfinder: **Kagabu, Shinzo, 432-131-105, Terada-machi, Hachioji-shi Tokyo (JP)**
Erfinder: **Sakawa, Shinji, 1-14-13, Tamadaira, Hino-shi Tokyo (JP)**

(74) Vertreter: **Schumacher, Günter, Dr. et al, c/o Bayer AG Konzernverwaltung RP Patentabteilung, D-5090 Leverkusen 1 Bayerwerk (DE)**

## (54) 2,2-Dichloro-(cis)-3-methyl-(trans)-3-chloro-methylcyclopropanmethanol.

(57) Die vorliegende Erfindung betrifft 2,2-Dichloro-(cis)-3-methyl-(trans)-3-chloromethylcyclopropanmethanol, eine neue Verbindung, die als Fungizid für Landwirtschaft und Gartenbau oder als Zwischenstufe für die Synthese anderer Cyclopropan-Derivate und anderer Verbindungen wertvoll ist. Die Verbindung kann beispielsweise mittels des folgenden Verfahrens hergestellt werden, in dem eine Verbindung der Formel (II)

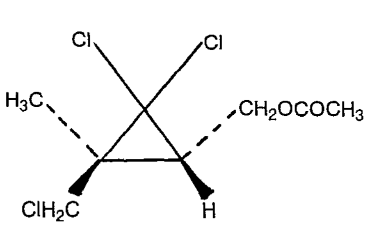

(II)

mit einem Alkalimetallhydroxid umgesetzt wird.

EP 0 135 897 A2

0135897

NIHON TOKUSHU NOYAKU SEIZO K.K., Tokyo, Japan

## 2,2-Dichloro-(cis)-3-methyl-(trans)-3-chloro-methylcyclopropanmethanol

Die vorliegende Erfindung betrifft 2,2-Dichloro-(cis)-3-methyl-(trans)-3-chloromethylcyclopropanmethanol, eine neue Verbindung, die als Fungizid für Landwirtschaft und Gartenbau oder als Zwischenstufe für die Synthese anderer Cyclopropan-Derivate und anderer Verbindungen wertvoll ist, sowie ein Verfahren zur Herstellung dieser Verbindung.

Im einzelnen betrifft die Erfindung 2,2-Dichloro-(cis)-3-methyl-(trans)-3-chloromethylcyclopropanmethanol der nachstehenden Formel (I)

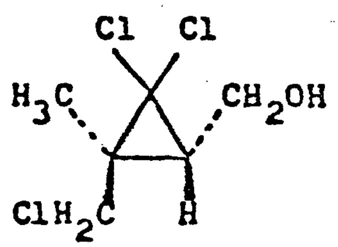

(I)

Die Verbindung der Formel (I) gemäß der vorliegenden Erfindung kann beispielsweise mit Hilfe des folgenden Verfahrens hergestellt werden, auf das sich die Erfindung ebenfalls erstreckt.

Ein Verfahren zur Herstellung von 2,2-Dichloro-(cis)-3-methyl-(trans)-3-chloromethylcyclopropanmethanol umfaßt die Reaktion einer Verbindung der nachstehenden Formel (II)

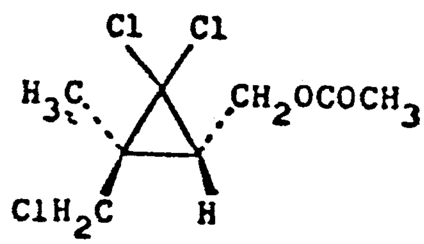

(II)

mit einem Alkalimetallhydroxid.

Untersuchungen seitens der Anmelderin haben ergeben, daß das vorstehende, in der bekannten Literatur nicht beschriebene 2,2-Dichloro-(cis)-3-methyl-(trans)-3-chloromethylcyclopropanmethanol synthetisiert werden kann und daß diese Verbindung als Zwischenprodukt für die Herstellung neuer Verbindungen mit biologischer Aktivität als Agrochemikalien, insbesondere beispielsweise einer Aktivität in bezug auf die Bekämpfung der Brusone-Krankheit von Reis, wie des 2,2-Dichloro-(cis)-3-methyl-(trans)-3-chloromethylcyclopropanmethanol-chloroacetats wertvoll ist.

Weiterhin wurde gefunden, daß die Verbindung selbst eine hervorragende Bekämpfungswirkung gegen die Brusone-Krankheit von Reis zeigt.

Dementsprechend ist es Ziel der vorliegenden Erfindung, 2,2-Dichloro-(cis)-3-methyl-(trans)-3-chloromethylcyclopropanmethanol, eine neue Verbindung, sowie ein Verfahren zu seiner Herstellung verfügbar zu machen.

Die Verbindung gemäß der vorliegenden Erfindung kann beispielsweise mit Hilfe des folgenden Verfahrens hergestellt werden.

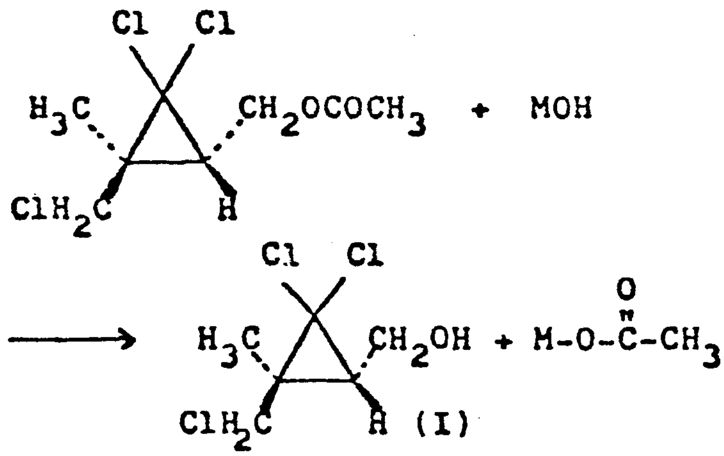

(II)

(I)

In dem vorstehenden Reaktionsschema steht M für ein Alkalimetall-Atom, beispielsweise Natrium oder Kalium.

Das Verfahren der vorliegenden Erfindung wird speziell durch das folgende Beispiel beschrieben:

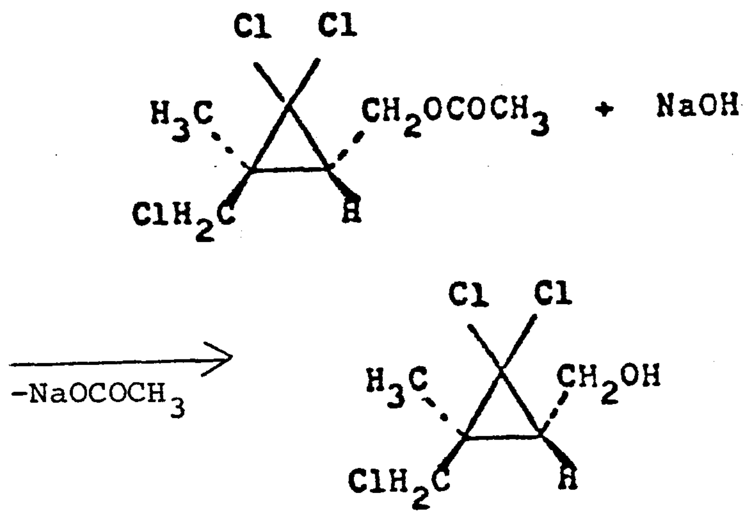


Zweckmäßigerweise kann das Verfahren zur Herstellung der vorstehenden Verbindung gemäß der vorliegenden

Erfindung unter Verwendung eines Lösungsmittels oder Verdünnungsmittels durchgeführt werden. Zu diesem Zweck können sämtliche inerten Lösungsmittel und Verdünnungsmittel verwendet werden.

Zu Beispielen für solche Lösungsmittel oder Verdünnungsmittel zählen Wasser; aliphatische, alicyclische und aromatische Kohlenwasserstoffe (ggf. chloriert) wie Hexan, Cyclohexan, Petrolether, Ligroin, Benzol, Toluol, Xylol, Methylenchlorid, Chloroform, Kohlenstofftetrachlorid, Ethylenchlorid, Trichloroethylen und Chlorbenzol; Ether wie Diethylether, Methylethylether, Diisopropylether, Dibutylether, Propylenoxid, Dioxan und Tetrahydrofuran; Ketone wie Aceton, Methylethylketon, Methylisopropylketon und Methylisobutylketon; Nitrile wie Acetonitril, Propionitril und Acrylnitril; Alkohole wie Methanol, Ethanol, Isopropanol, Butanol und Ethylenglycol; Ester wie Ethylacetat und Amylacetat; Säureamide wie Dimethylformamid und Dimethylacetamid; Sulfone und Sulfoxide wie Dimethylsulfoxid und Sulfolan; und Basen wie Pyridin.

Die obige Reaktion kann in Gegenwart eines säurebindenden Mittels durchgeführt werden. Zu Beispielen für das säurebindende Mittel zählen die Hydroxide, Carbonate, Hydrogencarbonate und Alkoholate von Alkalimetallen, die allgemein verwendet werden, und tertiäre Amine wie Triethylamin, Diethylanilin und Pyridin.

Das Verfahren gemäß der vorliegenden Erfindung kann in einem weiten Temperaturbereich durchgeführt werden. Im allgemeinen kann es bei einer Temperatur zwischen etwa -20°C und dem Siedepunkt der Mischung, vorzugsweise

zwischen etwa 0°C und etwa 100°C, durchgeführt werden. Die Reaktion wird zweckmäßigerweise unter Atmosphärendruck durchgeführt, jedoch ist es auch möglich, unter erhöhtem oder vermindertem Druck zu arbeiten.

Die folgenden Beispiele erläutern die vorliegende Erfindung. Es ist jedoch darauf hinzuweisen, daß die Erfindung nicht auf diese Beispiele allein beschränkt ist.

Beispiel 1 (Synthesebeispiel)

Eine Mischung aus 5 g 2,2-Dichloro-(cis)-3-methyl-(trans)-3-chloromethylcyclopropanmethanol-acetat, 50 ml Methanol und 5 ml einer 20-proz. wäßrigen Natriumhydroxid-Lösung wurde 5 h bei 50°C gerührt. Die Reaktionsmischung wurde abgekühlt und mit Diethylether extrahiert. Die etherische Schicht wurde getrocknet, und danach wurde der Ether abgedampft. Der Rückstand wurde destilliert, wonach 3,5 g des 2,2-Dichloro-(cis)-3-methyl-(trans)-3-chloromethylcyclopropanmethanols mit einem Sdp. von 148-150°C/30,7 mbar (23 mmHg), der gewünschten Verbindung der vorliegenden Erfindung, erhalten wurden.

Das folgende Bezugsbeispiel 1 erläutert die Synthese des 2,2-Dichloro-(cis)-3-methyl-(trans)-3-chloromethylcyclopropanmethanol-acetats, das als Ausgangsstoff in dem vorsteheden Verfahren gemäß der vorliegenden Erfindung eingesetzt wurde.

Bezugs-Synthesebeispiel 1

Natriumtrichloroacetat (45 g) wurde nach und nach im Laufe von 2 h unter Rühren bei 110°C zu 21,8 g trans-4-Chloro-3-methylcrotylacetat hinzugefügt. Nach der Zugabe wurde die Mischung weitere 2 h bei dieser Temperatur gerührt. Die Reaktionsmischung wurde abgekühlt, und 100 ml n-Hexan wurden zugegeben. Die Mischung wurde 10 min gerührt und dann über Nacht stehen gelassen. Der unlösliche Anteil wurde durch Filtration entfernt und mit 100 ml n-Hexan gewaschen, und danach wurde das Filtrat mit zwei Portionen von je 50 ml gesättigter wäßriger Natriumchloridlösung gewaschen und getrocknet. Das n-Hexan wurde abgedampft, und der Rückstand wurde bei 26,7 mbar (20 mmHg) destilliert, wobei die von 120°C bis 160°C siedenden Fraktionen abgetrennt wurden. Diese Fraktionen wurden nochmals bei 33,3 mbar (25 mmHg) fraktioniert destilliert, wonach 18,3 g 2,2-Dichloro-(cis)-3-methyl-(trans)-3-chloromethylcyclopropanmethanol-acetat mit einem Siedepunkt 152-154°C/33,3 mbar (25 mmHg) erhalten wurden.

Nachstehend angegeben ist das Bezugs-Synthesebeispiel 2 für die Herstellung einer neuen, biologisch aktiven Verbindung aus der Verbindung gemäß der vorliegenden Erfindung als Zwischenprodukt.

## Bezugs-Synthesebeispiel 2

2,0 g 2,2-Dichloro-(cis)-3-methyl-(trans)-3-chloromethylcyclopropanmethanol und 1,0 g Pyridin wurden in Toluol (30 ml) gelöst, und unter Rühren und Eiskühlung wurde eine Lösung von 1,2 g Chloroacetylchlorid in 10 ml Toluol tropfenweise hinzugefügt. Die Mischung wurde 8 h gerührt und dann in Wasser gegossen. Die Toluol-Schicht wurde mit einer 3-proz. wäßrigen Salzsäure-Lösung, einer 10-proz. wäßrigen Natriumhydrogencarbonat-Lösung und danach mit Wasser gewaschen und dann

getrocknet. Das Toluol wurde unter vermindertem Druck abgedampft. Der Rückstand unter vermindertem Druck destilliert, wonach 2,6 g 2,2-Dichloro-(cis)-3-methyl-(trans)-3-chloromethylcyclopropanmethanol-chloroacetat mit einem Siedepunkt von 123-124°C/0,53 mbar (0,4 mmHg) der nachstehenden Formel erhalten wurden.

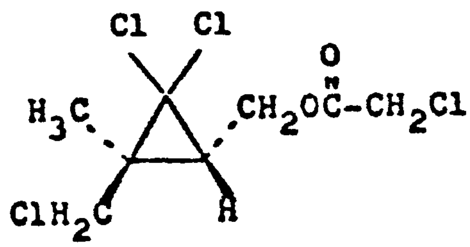

(D-1)

## Biologisches Test-Beispiel

Test auf Wirksamkeit gegen die Brusone-Krankheit von Reis bei Wasseroberflächen-Anwendung:

Herstellung einer Test-Verbindung:

| | |
|---|---|
| Wirkstoff: | 50 Gew.-Teile; |
| Träger: | 45 Gew.-Teile einer Mischung (1 : 5) aus Diatomeenerde und Kaolin; |
| Emulgator: | 5 Gew.-Teile Polyoxyethylenalkylphenylether. |

Die aktive Verbindung, der Träger und der Emulgator wurden pulverisiert und in den angegebenen Mengen miteinander vermischt, wodurch ein benetzbares Pulver gebildet wurde. Eine vorher festgelegte Menge des benetzbaren Pulvers wurde mit Wasser verdünnt, wodurch ein Test-Präparat hergestellt wurde.

Test-Verfahren

Reispflanzen (Varietät: Asahi) wurden in überfluteten Porzellan-Töpfen mit einem Durchmesser von 12 cm, jeweils drei Setzlinge pro Topf, kultiviert. Im Frühstadium des Austreibens der Reispflanzen wurde die wie oben auf eine vorher festgelegte Konzentration gebrachte Test-Verbindung in den angegebenen Dosierungen mit Hilfe einer Pipette auf die Wasseroberfläche aufgebracht, so daß sie nicht unmittelbar mit den oberirdischen Teilen der Reispflanzen in Berührung kam. Vier Tage später wurde eine Suspension von Sporen des Brusone-Pilzes (Piricularia oryzae) in üblicher Weise auf die Reispflanzen gesprüht, um sie mit dem Pilz zu inokulieren. Die Pflanzen wurden 24 h in einem Inkubator bei 23-25°C und einer relativen Luftfeuchtigkeit von 100 % gehalten. Danach wurden sie in ein auf 20-28°C gehaltenes Glas-Gewächshaus überführt. Sieben Tage nach der Beimpfung wurden die Pflanzen untersucht und aufgrund des folgenden Bewertungsmaßstabs beurteilt. Der Bekämpfungs-Index (%) wurde berechnet.

| Grad der Erkrankung | Fläche der Verletzungen (%) |
|---|---|
| 0 | 0 |
| 0,5 | 2 oder weniger |
| 1 | 3 bis 5 |
| 2 | 6 bis 10 |
| 3 | 11 bis 20 |
| 4 | 21 bis 40 |
| 5 | 41 oder mehr |

$$\text{Bekämpfungs-Index (\%)} = \frac{\text{Grad der Erkrankung einer unbehandelten Fläche} - \text{Grad der Erkrankung einer behandelten Fläche}}{\text{Grad der Erkrankung einer unbehandelten Fläche}} \times 100$$

Die Ergebnisse sind in der Tabelle 1 aufgeführt. Diese Ergebnisse wurden aus drei Töpfen pro Prüfung erhalten.

<u>Tabelle 1</u>

| Beispiel Nr. | Wirkstoff-Konzentration $g/m^2$ | Bekämpfungs-Index (%) | Phytotoxizität |
|---|---|---|---|
| Verbindung d. Erfindung | 0,8 | 100 | - |
| D-1 | 0,8 | 100 | - |

Anmerkungen zu Tabelle 1:

(1) Die Verbindung gemäß der vorliegenden Erfindung ist

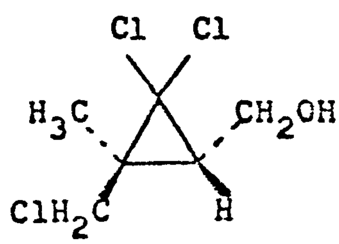

(2) Die Verbindung D-1 (Derivat) ist

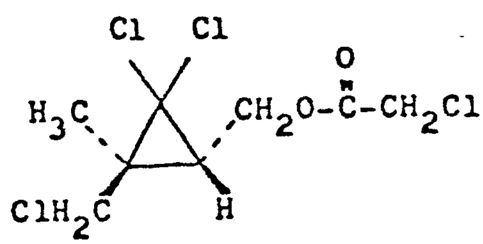

(3) Das Zeichen "-" in der Spalte der Phytotoxizität gibt an, daß keine Phytotoxizität vorlag.

Die in der vorliegenden Schrift beschrieben Erfindung wird wie folgt zusammengefaßt:

(1) 2,2-Dichloro-(cis)-3-methyl-(trans)-3-chloromethylcyclopropanmethanol.

(2) Verfahren zur Herstellung von 2,2-Dichloro-(cis)-3-methyl-(trans)-3-chloromethylcyclopropanmethanol, bei dem eine Verbindung der Formel

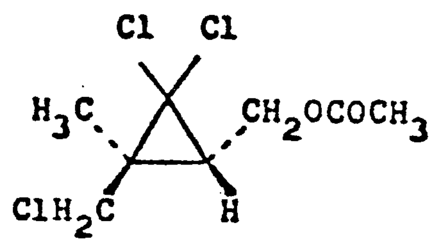

mit einem Alkalimetallhydroxid umgesetzt wird.

0135897

## Patentansprüche

1. 2,2-Dichloro-(cis)-3-methyl-(trans)-3-chloromethyl-cyclopropanmethanol der Formel (I)

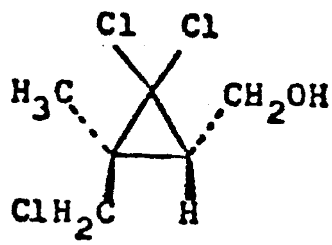

(I)

.

2. Verfahren zur Herstellung von 2,2-Dichloro-(cis)-3-methyl-(trans)-3-chloromethylcyclopropanmethanol der Formel (I),

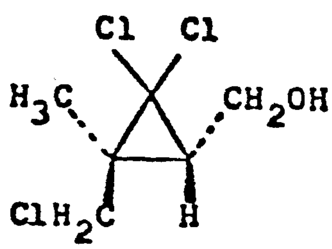

(I)

,

dadurch gekennzeichnet, daß eine Verbindung der Formel (II)

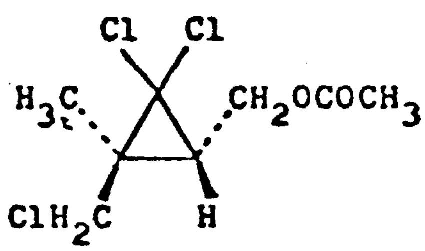

(II)

mit einem Alkalimetallhydroxid, gegebenenfalls in Gegenwart eines Verdünnungsmittels, umgesetzt wird.